# EUROPEAN PATENT APPLICATION

(11) **EP 2 078 726 A1**
(43) Date of publication of application: **15.07.2009**
(21) Application number: 08000298.3
(22) Date of filing: 09.01.2008
(51) Int. Cl.: C07K 14/16, A61K 48/00, C12N 15/867, A61P 31/18

(54) **Secretable HIV entry inhibitory peptides for therapy of HIV infection**

(71) Applicant: Vision 7 GmbH, 60596 Frankfurt am Main (DE); Chemotherapeutisches Forschungsinstitut Georg-Speyer-Haus, 60596 Frankfurt (DE)
(72) Inventor: von Laer, Dorothee, Prof. Dr., 60596 Frankfurt am Main (DE); Egerer, Lisa, 60385 Frankfurt am Main (DE)
(74) Representative: UEXKÜLL & STOLBERG

(57) **Abstract**

The invention relates to the treatment of an HIV infection by the expression of in vivo secretable peptides that prevent entry of the virus into the cell. The invention makes available nucleic acids and vectors containing the same that code for a fusion protein that contains two HIV entry inhibitory peptides connected by a cleavable or non-cleavable, flexible linker.

SEQUENCE LISTING

<110>  Vision-7 GmbH and Chemotherapeutisches Forschungsinstitut
       Georg-Speyer-Haus

<120>  Secretable HIV entry inhibitory peptides for therapy of HIV
       infection

<130>  P 76460

<160>  32

<170>  PatentIn version 3.4

<210>  1
<211>  23
<212>  PRT
<213>  Homo sapiens

<400>  1

Met Asp Ala Met Lys Arg Gly Leu Cys Cys Val Leu Leu Leu Cys Gly
1               5                   10                  15

Ala Val Phe Val Ser Pro Ser
            20

<210>  2
<211>  28
<212>  PRT
<213>  Homo sapiens

<400>  2

Met Gly Ala Gly Ala Thr Gly Arg Ala Met Asp Gly Pro Arg Leu Leu
1               5                   10                  15

Leu Leu Leu Leu Leu Gly Val Ser Leu Gly Gly Ala
            20                  25

<210>  3
<211>  21
<212>  PRT
<213>  Mus musculus

<400>  3

Met Glu Thr Asp Thr Leu Leu Leu Trp Val Leu Leu Leu Trp Val Pro
1               5                   10                  15

Gly Ser Thr Gly Asp
            20

<210>  4
<211>  20
<212>  PRT
<213>  Human immunodeficiency virus type 1

<400>  4

Tyr Thr Ser Leu Ile His Ser Leu Ile Glu Glu Ser Gln Asn Gln Gln
1               5                   10                  15

Glu Lys Asn Glu
            20

<210>  5
<211>  46
<212>  PRT
<213>  Human immunodeficiency virus type 1

<400>  5

Trp Met Glu Trp Asp Arg Glu Ile Asn Asn Tyr Thr Ser Leu Ile His
1               5                   10                  15

Ser Leu Ile Glu Glu Ser Gln Asn Gln Gln Glu Lys Asn Glu Gln Glu
            20                  25                  30

Leu Leu Glu Leu Asp Lys Trp Ala Ser Leu Trp Asn Trp Phe
        35                  40                  45

<210>  6
<211>  46
<212>  PRT
<213>  Artificial sequence

<220>
<223>  C46mutGlyco

<400>  6

Trp Met Glu Trp Asp Arg Glu Ile Asn Asn Tyr Ala Ser Leu Ile His
1               5                   10                  15

Ser Leu Ile Glu Glu Ser Gln Asn Gln Gln Glu Lys Asn Glu Gln Glu
            20                  25                  30

Leu Leu Glu Leu Asp Lys Trp Ala Ser Leu Trp Asn Trp Phe
        35                  40                  45

<210>  7
<211>  34
<212>  PRT
<213>  Human immunodeficiency virus type 1

<400>  7

Trp Met Glu Trp Asp Arg Glu Ile Asn Asn Tyr Thr Ser Leu Ile His
1               5                   10                  15

Ser Leu Ile Glu Glu Ser Gln Asn Gln Gln Glu Lys Asn Glu Gln Glu
            20                  25                  30

Leu Leu

<210>  8
<211>  36

<212>  PRT
<213>  Human immunodeficiency virus type 1

<400>  8

Tyr Thr Ser Leu Ile His Ser Leu Ile Glu Glu Ser Gln Asn Gln Gln
1               5                   10                  15

Glu Lys Asn Glu Gln Glu Leu Leu Glu Leu Asp Lys Trp Ala Ser Leu
            20                  25                  30

Trp Asn Trp Phe
        35

<210>  9
<211>  39
<212>  PRT
<213>  Artificial sequence

<220>
<223>  T-1249

<400>  9

Trp Gln Glu Trp Glu Gln Lys Ile Thr Ala Leu Leu Glu Gln Ala Gln
1               5                   10                  15

Ile Gln Gln Glu Lys Asn Glu Tyr Glu Leu Gln Lys Leu Asp Lys Trp
            20                  25                  30

Ala Ser Leu Trp Glu Trp Phe
        35

<210>  10
<211>  36
<212>  PRT
<213>  Artificial sequence

<220>
<223>  T-649

<400>  10

Trp Met Glu Trp Asp Arg Glu Ile Asn Asn Tyr Thr Ser Leu Ile His
1               5                   10                  15

Ser Leu Ile Glu Glu Ser Gln Asn Gln Gln Glu Lys Asn Glu Gln Glu
            20                  25                  30

Leu Leu Glu Leu
        35

<210>  11
<211>  35
<212>  PRT
<213>  Artificial sequence

<220>
<223>  SC35EK

<400>  11

Trp Glu Glu Trp Asp Lys Lys Ile Glu Glu Tyr Thr Lys Lys Ile Glu
1               5                   10                  15

Glu Leu Ile Lys Lys Ser Glu Glu Gln Gln Lys Lys Asn Glu Glu Glu
            20                  25                  30

Leu Lys Lys
        35

<210>  12
<211>  38
<212>  PRT
<213>  Artificial sequence

<220>
<223>  T-2635

<400>  12

Thr Thr Trp Glu Ala Trp Asp Arg Ala Ile Ala Glu Tyr Ala Ala Arg
1               5                   10                  15

Ile Glu Ala Leu Ile Arg Ala Ala Gln Glu Gln Gln Glu Lys Asn Glu
            20                  25                  30

Ala Ala Leu Arg Glu Leu
        35

<210>  13
<211>  46
<212>  PRT
<213>  Human immunodeficiency virus type 2

<400>  13

Trp Gln Gln Trp Glu Arg Gln Val Arg Phe Leu Asp Ala Asn Ile Thr
1               5                   10                  15

Lys Leu Leu Glu Glu Ala Gln Ile Gln Gln Glu Lys Asn Met Tyr Glu
            20                  25                  30

Leu Gln Glu Leu Asp Lys Trp Ala Ser Leu Trp Asn Trp Phe
        35                  40                  45

<210>  14
<211>  4
<212>  PRT
<213>  Homo sapiens

<400>  14

Arg Ala Lys Arg
1

<210>  15

<211>  4
<212>  PRT
<213>  Homo sapiens

<400>  15

Arg Gly Arg Arg
1


<210>  16
<211>  8
<212>  PRT
<213>  Artificial sequence

<220>
<223>  Furin opt

<400>  16

Arg Ser Arg Ala Lys Arg Ser Val
1               5


<210>  17
<211>  8
<212>  PRT
<213>  Artificial sequence

<220>
<223>  Furin GG

<400>  17

Gly Gly Arg Gly Arg Arg Gly Gly
1               5


<210>  18
<211>  19
<212>  PRT
<213>  Human immunodeficiency virus type 1

<400>  18

Pro Thr Lys Ala Lys Arg Arg Val Val Gln Arg Glu Lys Arg Ala Val
1               5                   10                  15


Gly Ile Gly



<210>  19
<211>  4
<212>  PRT
<213>  Homo sapiens

<400>  19

Arg Arg Leu Leu
1


<210>  20
<211>  19
<212>  PRT
<213>  Homo sapiens

<400>  20

Glu Arg Lys Cys Cys Val Glu Cys Pro Pro Cys Pro Ala Pro Pro Val
1               5                   10                  15

Ala Gly Pro

<210>  21
<211>  12
<212>  PRT
<213>  Human immunodeficiency virus type 1

<400>  21

Asn Ile Thr Asn Trp Leu Trp Tyr Ile Lys Leu Phe
1               5                   10

<210>  22
<211>  10
<212>  PRT
<213>  Artificial sequence

<220>
<223>  (G4S)2

<400>  22

Gly Gly Gly Gly Ser Gly Gly Gly Gly Ser
1               5                   10

<210>  23
<211>  20
<212>  PRT
<213>  Artificial sequence

<220>
<223>  (G4S)4

<400>  23

Gly Gly Gly Gly Ser Gly Gly Gly Gly Ser Gly Gly Gly Gly Ser Gly
1               5                   10                  15

Gly Gly Gly Ser
            20

<210>  24
<211>  69
<212>  PRT
<213>  Artificial sequence

<220>
<223>  S-C46

<220>
<221>  SP-tPA
<222>  (1)..(23)

<220>

<221>  HIV-1 gp41 C46
<222>  (24)..(69)

<400>  24

Met Asp Ala Met Lys Arg Gly Leu Cys Cys Val Leu Leu Leu Cys Gly
1               5                   10                  15

Ala Val Phe Val Ser Pro Ser Trp Met Glu Trp Asp Arg Glu Ile Asn
            20                  25                  30

Asn Tyr Thr Ser Leu Ile His Ser Leu Ile Glu Glu Ser Gln Asn Gln
        35                  40                  45

Gln Glu Lys Asn Glu Gln Glu Leu Leu Glu Leu Asp Lys Trp Ala Ser
    50                  55                  60

Leu Trp Asn Trp Phe
65


<210>  25
<211>  99
<212>  PRT
<213>  Artificial sequence

<220>
<223>  S-C46-HIVLinker-myc


<220>
<221>  SP-tPA
<222>  (1)..(23)

<220>
<221>  HIV-1 gp41 C46
<222>  (24)..(69)

<220>
<221>  HIVLinker
<222>  (70)..(87)

<220>
<221>  cMyc tag
<222>  (88)..(97)

<400>  25

Met Asp Ala Met Lys Arg Gly Leu Cys Cys Val Leu Leu Leu Cys Gly
1               5                   10                  15

Ala Val Phe Val Ser Pro Ser Trp Met Glu Trp Asp Arg Glu Ile Asn
            20                  25                  30

Asn Tyr Thr Ser Leu Ile His Ser Leu Ile Glu Glu Ser Gln Asn Gln
        35                  40                  45

Gln Glu Lys Asn Glu Gln Glu Leu Leu Glu Leu Asp Lys Trp Ala Ser
    50                  55                  60

Leu Trp Asn Trp Phe Arg Ser Asn Ile Thr Asn Trp Leu Trp Tyr Ile
65                  70                  75                  80

Lys Leu Phe Glu Phe Leu Gln Glu Gln Lys Leu Ile Ser Glu Glu Asp
                85                  90                  95

Leu Leu Gln

<210>  26
<211>  120
<212>  PRT
<213>  Artificial sequence

<220>
<223>  S-C46-HIVLinker-hIgG2Linker-myc

<220>
<221>  SP-tPA
<222>  (1)..(23)

<220>
<221>  HIV-1 gp41 C46
<222>  (24)..(69)

<220>
<221>  HIVLinker
<222>  (70)..(85)

<220>
<221>  hIgG2Linker
<222>  (86)..(106)

<220>
<221>  cMyc tag
<222>  (107)..(116)

<400>  26

Met Asp Ala Met Lys Arg Gly Leu Cys Cys Val Leu Leu Leu Cys Gly
1               5                   10                  15

Ala Val Phe Val Ser Pro Ser Trp Met Glu Trp Asp Arg Glu Ile Asn
            20                  25                  30

Asn Tyr Thr Ser Leu Ile His Ser Leu Ile Glu Glu Ser Gln Asn Gln
        35                  40                  45

Gln Glu Lys Asn Glu Gln Glu Leu Leu Glu Leu Asp Lys Trp Ala Ser
    50                  55                  60

Leu Trp Asn Trp Phe Arg Ser Asn Ile Thr Asn Trp Leu Trp Tyr Ile
65                  70                  75                  80

Lys Leu Phe Glu Leu Glu Arg Lys Cys Cys Val Glu Cys Pro Pro Cys
                85                  90                  95

Pro Ala Pro Pro Val Ala Gly Pro Glu Leu Glu Gln Lys Leu Ile Ser

            100                 105                 110

Glu Glu Asp Leu Glu Phe Leu Gln
        115                 120

<210> 27
<211> 126
<212> PRT
<213> Artificial sequence

<220>
<223> S-2xC46-Furin mut

<220>
<221> SP-tPA
<222> (1)..(23)

<220>
<221> HIV-1 gp41 C46
<222> (24)..(69)

<220>
<221> Furin mut
<222> (70)..(74)

<220>
<221> HIV-1 gp41 C46
<222> (75)..(120)

<400> 27

Met Asp Ala Met Lys Arg Gly Leu Cys Cys Val Leu Leu Leu Cys Gly
1               5                   10                  15

Ala Val Phe Val Ser Pro Ser Trp Met Glu Trp Asp Arg Glu Ile Asn
            20                  25                  30

Asn Tyr Thr Ser Leu Ile His Ser Leu Ile Glu Glu Ser Gln Asn Gln
        35                  40                  45

Gln Glu Lys Asn Glu Gln Glu Leu Leu Glu Leu Asp Lys Trp Ala Ser
    50                  55                  60

Leu Trp Asn Trp Phe Arg Ala Lys Val Asp Trp Met Glu Trp Asp Arg
65                  70                  75                  80

Glu Ile Asn Asn Tyr Thr Ser Leu Ile His Ser Leu Ile Glu Glu Ser
                85                  90                  95

Gln Asn Gln Gln Glu Lys Asn Glu Gln Glu Leu Leu Glu Leu Asp Lys
            100                 105                 110

Trp Ala Ser Leu Trp Asn Trp Phe Arg Arg Leu Leu Asp Ile
        115                 120                 125

<210> 28
<211> 126

<212>   PRT
<213>   Artificial sequence

<220>
<223>   S-2xC46-Furin


<220>
<221>   SP-tPA
<222>   (1)..(23)

<220>
<221>   HIV-1 gp41 C46
<222>   (24)..(69)

<220>
<221>   Furin
<222>   (70)..(74)

<220>
<221>   HIV-1 gp41 C46
<222>   (75)..(120)

<400>   28

Met Asp Ala Met Lys Arg Gly Leu Cys Cys Val Leu Leu Leu Cys Gly
1               5                   10                  15


Ala Val Phe Val Ser Pro Ser Trp Met Glu Trp Asp Arg Glu Ile Asn
            20                  25                  30


Asn Tyr Thr Ser Leu Ile His Ser Leu Ile Glu Glu Ser Gln Asn Gln
        35                  40                  45


Gln Glu Lys Asn Glu Gln Glu Leu Leu Glu Leu Asp Lys Trp Ala Ser
    50                  55                  60


Leu Trp Asn Trp Phe Arg Ala Lys Arg Asp Trp Met Glu Trp Asp Arg
65                  70                  75                  80


Glu Ile Asn Asn Tyr Thr Ser Leu Ile His Ser Leu Ile Glu Glu Ser
                85                  90                  95


Gln Asn Gln Gln Glu Lys Asn Glu Gln Glu Leu Leu Glu Leu Asp Lys
            100                 105                 110


Trp Ala Ser Leu Trp Asn Trp Phe Arg Arg Leu Leu Asp Ile
        115                 120                 125


<210>   29
<211>   129
<212>   PRT
<213>   Artificial sequence

<220>
<223>   S-2xC46-Furin opt


<220>
<221>   SP-tPA

<222>  (1)..(23)

<220>
<221>  HIV-1 gp41 C46
<222>  (24)..(69)

<220>
<221>  Furin opt
<222>  (70)..(77)

<220>
<221>  HIV-1 gp41 C46
<222>  (78)..(123)

<400>  29

Met Asp Ala Met Lys Arg Gly Leu Cys Cys Val Leu Leu Leu Cys Gly
1               5                   10                  15

Ala Val Phe Val Ser Pro Ser Trp Met Glu Trp Asp Arg Glu Ile Asn
            20                  25                  30

Asn Tyr Thr Ser Leu Ile His Ser Leu Ile Glu Glu Ser Gln Asn Gln
        35                  40                  45

Gln Glu Lys Asn Glu Gln Glu Leu Leu Glu Leu Asp Lys Trp Ala Ser
    50                  55                  60

Leu Trp Asn Trp Phe Arg Ser Arg Ala Lys Arg Ser Val Trp Met Glu
65                  70                  75                  80

Trp Asp Arg Glu Ile Asn Asn Tyr Thr Ser Leu Ile His Ser Leu Ile
                85                  90                  95

Glu Glu Ser Gln Asn Gln Gln Glu Lys Asn Glu Gln Glu Leu Leu Glu
            100                 105                 110

Leu Asp Lys Trp Ala Ser Leu Trp Asn Trp Phe Arg Arg Leu Leu Asp
        115                 120                 125

Ile

<210>  30
<211>  123
<212>  PRT
<213>  Artificial sequence

<220>
<223>  S-2xC46-Furin opt 1

<220>
<221>  SP-tPA
<222>  (1)..(23)

<220>
<221>  HIV-1 gp41 C46
<222>  (24)..(69)

<220>
<221>  Furin opt
<222>  (70)..(77)

<220>
<221>  HIV-1 gp41 C46
<222>  (78)..(123)

<400>  30

Met Asp Ala Met Lys Arg Gly Leu Cys Cys Val Leu Leu Leu Cys Gly
1               5                   10                  15

Ala Val Phe Val Ser Pro Ser Trp Met Glu Trp Asp Arg Glu Ile Asn
            20                  25                  30

Asn Tyr Thr Ser Leu Ile His Ser Leu Ile Glu Glu Ser Gln Asn Gln
        35                  40                  45

Gln Glu Lys Asn Glu Gln Glu Leu Leu Glu Leu Asp Lys Trp Ala Ser
    50                  55                  60

Leu Trp Asn Trp Phe Arg Ser Arg Ala Lys Arg Ser Val Trp Met Glu
65                  70                  75                  80

Trp Asp Arg Glu Ile Asn Asn Tyr Thr Ser Leu Ile His Ser Leu Ile
                85                  90                  95

Glu Glu Ser Gln Asn Gln Gln Glu Lys Asn Glu Gln Glu Leu Leu Glu
            100                 105                 110

Leu Asp Lys Trp Ala Ser Leu Trp Asn Trp Phe
        115                 120

<210>  31
<211>  372
<212>  DNA
<213>  Artificial sequence

<220>
<223>  Nucleotide sequence of S-2xC46-Furin opt 1

<400>  31
atggatgcaa tgaagagagg gctctgctgt gtgctgctgc tgtgtggagc agtcttcgtt      60

tcgcccagct ggatggagtg ggaccgcgag atcaacaact acaccagcct gatccacagc     120

ctgatcgagg agagccagaa ccagcaggag aagaacgagc aggagctgct ggagctggac     180

aagtgggcca gcctgtggaa ctggttcagg agccgcgcca agaggagcgt gtggatggag     240

tgggaccgcg agattaacaa ctacaccagc ctgatccaca gcctgatcga ggagagccag     300

aaccagcagg agaagaacga gcaggagctg ctggagctgg acaagtgggc cagcctgtgg     360

aactggttct ag                                                         372

<210>  32

<211>  345
<212>  PRT
<213>  Human immunodeficiency virus type 1

<400>  32

Ala Val Gly Ile Gly Ala Leu Phe Leu Gly Phe Leu Gly Ala Ala Gly
1               5                   10                  15

Ser Thr Met Gly Ala Ala Ser Met Thr Leu Thr Val Gln Ala Arg Gln
            20                  25                  30

Leu Leu Ser Gly Ile Val Gln Gln Gln Asn Asn Leu Leu Arg Ala Ile
        35                  40                  45

Glu Ala Gln Gln His Leu Leu Gln Leu Thr Val Trp Gly Ile Lys Gln
    50                  55                  60

Leu Gln Ala Arg Ile Leu Ala Val Glu Arg Tyr Leu Lys Asp Gln Gln
65                  70                  75                  80

Leu Leu Gly Ile Trp Gly Cys Ser Gly Lys Leu Ile Cys Thr Thr Ala
                85                  90                  95

Val Pro Trp Asn Ala Ser Trp Ser Asn Lys Ser Leu Glu Gln Ile Trp
            100                 105                 110

Asn His Thr Thr Trp Met Glu Trp Asp Arg Glu Ile Asn Asn Tyr Thr
        115                 120                 125

Ser Leu Ile His Ser Leu Ile Glu Glu Ser Gln Asn Gln Gln Glu Lys
    130                 135                 140

Asn Glu Gln Glu Leu Leu Glu Leu Asp Lys Trp Ala Ser Leu Trp Asn
145                 150                 155                 160

Trp Phe Asn Ile Thr Asn Trp Leu Trp Tyr Ile Lys Leu Phe Ile Met
                165                 170                 175

Ile Val Gly Gly Leu Val Gly Leu Arg Ile Val Phe Ala Val Leu Ser
            180                 185                 190

Ile Val Asn Arg Val Arg Gln Gly Tyr Ser Pro Leu Ser Phe Gln Thr
        195                 200                 205

His Leu Pro Thr Pro Arg Gly Pro Asp Arg Pro Glu Gly Ile Glu Glu
    210                 215                 220

Glu Gly Gly Glu Arg Asp Arg Asp Arg Ser Ile Arg Leu Val Asn Gly
225                 230                 235                 240

Ser Leu Ala Leu Ile Trp Asp Asp Leu Arg Ser Leu Cys Leu Phe Ser
                245                 250                 255

Tyr His Arg Leu Arg Asp Leu Leu Leu Ile Val Thr Arg Ile Val Glu
            260                 265                 270

Leu Leu Gly Arg Arg Gly Trp Glu Ala Leu Lys Tyr Trp Trp Asn Leu
        275                 280                 285

Leu Gln Tyr Trp Ser Gln Glu Leu Lys Asn Ser Ala Val Ser Leu Leu
    290                 295                 300

Asn Ala Thr Ala Ile Ala Val Ala Glu Gly Thr Asp Arg Val Ile Glu
305                 310                 315                 320

Val Val Gln Gly Ala Cys Arg Ala Ile Arg His Ile Pro Arg Arg Ile
                325                 330                 335

Arg Gln Gly Leu Glu Arg Ile Leu Leu
            340                 345

## Description

Within the last decades HIV-1 (human immunodeficiency virus type 1) infection spread around the globe, so that today more than 40 million people throughout the world are infected by the virus. HIV-1 infection leads to a substantial loss of CD4+ T lymphocytes, resulting in the destruction of the cellular immune system and ultimately in the development of acquired immunodeficiency syndrome (AIDS).

Antiviral drug therapy can effectively suppress HIV-1 replication, and thereby preserve immune functions and prolong survival of HIV-infected patients, but drug toxicity and viral resistance limit the long-term therapeutic efficacy. Thus, as a vaccine is not in sight, novel therapeutic approaches are urgently needed.

Gene therapy is a valuable alternative in the treatment of HIV-1 infection. In any event, it might play an important supporting role to pharmacotherapy. Several different transgenes have been reported to inhibit HIV-1 *in vitro.* The successful and safe transfer of these therapeutic genes into autologous T cells or hematopoietic stem cells has already been proven in clinical trials. Long-term engraftment of gene-modified cells has been reported for both approaches. However, in vivo levels of gene marking were not sufficient to achieve a clinical benefit for the patients (reviewed in von Laer et al., J Gene Med 2006 and von Laer et al., CMC-AIAA 2007).

In general, gene therapeutic strategies for HIV-1 infection either target the specific elimination of infected cells or the protection of HIV-1 target cells from infection by the virus (so-called intracellular immunization). The major problem of intracellular immunization approaches is the huge number of potential HIV-1 target cells (10¹¹) in the human body that cannot be genetically modified with the available technologies. Hence, a therapeutic effect can only be achieved when the gene-protected cells have a selective advantage and therefore accumulate in vivo. As a result of mathematical modelling and computer simulations it became evident that merely so-called class I genes, which inhibit early steps in the HIV-1 replication cycle, could possibly fulfil this criterion (von Laer et al., J Theor Biol 2006). Class I genes are active prior to the integration of the proviral cDNA into the host cell genome, thereby preventing infection of the cell and thus providing a selective advantage. Surprisingly, only few early inhibitors for HIV-1 gene therapy are currently available.

The inventors previously developed a broadly active class I transgene for HIV-1 gene therapy targeting the viral entry mechanism. The HIV entry inhibitor maC46 is a membrane-anchored peptide derived from the second heptad repeat of the HIV-1 transmembrane glycoprotein gp41. Membrane-anchored C46 effectively inhibits fusion of the viral and cellular membranes during virus entry. The therapeutic transgene encoding maC46 is provided by the gamma-retroviral vector M87o and prevents viral replication in cell lines and primary blood lymphocytes when expressed on the cell surface. Furthermore, in HIV-infected cell cultures, maC46-expressing T cells have a strong selective advantage compared to non-modified cells, rapidly accumulate and gain prevalence, without carrying an integrated provirus (Egelhofer et al., J Virol 2004). Thus, this gene therapy approach was promising to possibly allow for gene-protected non-infected T cells to accumulate in a patient over time.

Therefore, the retroviral vector M87o was used for the genetic ex vivo modification of autologous T cells from AIDS patients in a clinical trial. However, although CD4 T cell counts transiently rose significantly after infusion of the gene-protected cells, the viral load was not affected. Accumulation of the gene-protected cells was not observed and thus levels of gene marking were too low to result in an overall clinical benefit (van Lunzen et al., Mol Ther 2007).

A way to overcome the observed deficiency and to provide a therapeutic effect despite low levels of gene marking could be the implementation of a secretory principle. Secretory antiviral gene products could exert a bystander effect on non-modified neighbouring cells, and as a result cause a therapeutic effect even at relatively low levels of gene marking. However, the known and most potent HIV-1 fusion inhibitors are small peptides composed of only 34-46 amino acids. The peptide size represents a major challenge for the development of a secretable entry inhibitory peptide, as cells usually secrete small peptides very inefficiently or not at all: to enter the secretory pathway proteins must exceed a certain minimal size of ∼70-80 amino acids (Eskridge & Shields, J Biol Chem 1983, Lipp et al., J Biol Chem 1987). In fact, secretory peptides less than to 70 amino acids long are common, but they always have to be cleaved from larger precursor proteins to circumvent the size restrictions for the translocation into the ER which initiates the secretory pathway.

Consequently, a need exists for an improved gene therapeutic strategy that would overcome the aforementioned disadvantages.

Within the scope of the present invention, it has now surprisingly been found that retroviral vectors can be designed that encode in *vivo* secretable HIV entry inhibitory peptides. Secretion of these peptides by cells transduced with the retroviral vector is supposed to protect the gene-modified cells as well as non-modified neighbouring cells from HIV infection.

### SUMMARY OF THE INVENTION

The subject of the present invention is a retroviral vector that encodes secretable HIV entry inhibitory peptides, for the therapy of the human immunodeficiency virus (HIV) infection.

Accordingly, in one aspect the invention relates to a nucleic acid of the general formula
5' - SP - EI1 - LINKER - EI2 - 3',
wherein
- 5': designates the 5' end of the nucleic acid sequence,
- 3': designates the 3' end of the nucleic acid sequence,
- SP: encodes a signal peptide,
- EI1: encodes an HIV entry inhibitory peptide,
- EI2: encodes an HIV entry inhibitory peptide, and
- LINKER: encodes a linker between EI1 and EI2; and
wherein the linker is a cleavable linker or a non-cleavable, flexible linker.

In another aspect, the invention relates to a vector containing said nucleic acid. The vector of the invention is any vector suitable for delivering the nucleic acid of the invention into cells by means of transduction or transfection. For example, the vector is a retroviral vector, a lentiviral vector, an Epstein-Barr virus (EBV) vector, a self-inactivating vector (SIN vector), a non-integrating viral vector such as an adenoviral vector, or a non-viral vector such as a plasmid.

In a further aspect, the invention relates to cells transduced or transfected with the vector of the invention. The cells are cells of the lymphocyte lineage such as B and T cells, haematopoietic stem or progenitor cells, or mesenchymal stem or stromal cells.

In a further aspect, the invention relates to a method for in vitro transfection of cells of the invention with the vector of the invention.

In a further aspect, the invention relates to the use of the vector of the invention for the preparation of a pharmaceutical composition for the treatment of patients infected with the human immunodeficiency virus.

In a further aspect, the invention relates to the use of the cells of the invention for the preparation of a pharmaceutical composition for the treatment of patients infected with the human immunodeficiency virus.

### DESCRIPTION OF THE FIGURES

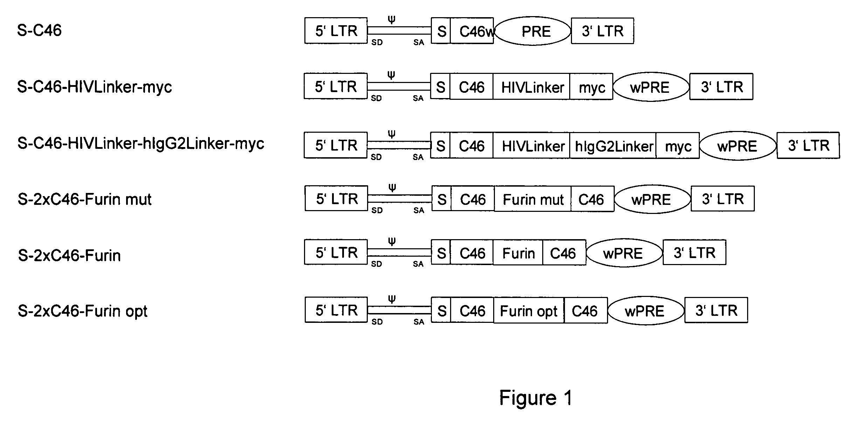

Figure 1: Structure of the retroviral vectors used. LTR, long terminal repeat; S, signal peptide; SA, splice acceptor; SD, splice donor; Ψ, packaging signal; wPRE, woodchuck hepatitis virus posttranscriptional regulatory element.

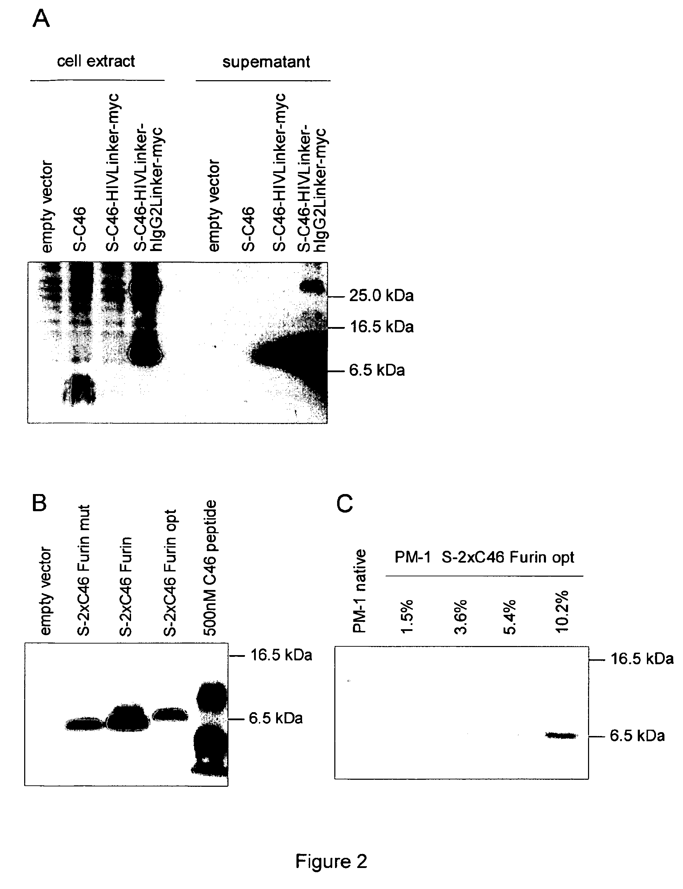

Figure 2: Inhibitory peptides are secreted by transfected and transduced cells. (A, B) Western blot of peptides secreted by transfected HEK 293T cells. Cells were transfected with retroviral expression vectors encoding the indicated secretable peptides, or empty vector. Cell culture supernatants and cell extracts were produced and analyzed by Western blot after de-glycosylation. Synthetically produced C46 peptide was loaded as standard to make a rough estimate of peptide concentrations. In (A) 5 µl of cell extracts (corresponding to 2.5x10⁵ cells) or 10 µl of cell culture supernatants were loaded per lane. In (B) 8 µl deglycosylated supernatant or C46 peptide solution of indicated concentration was loaded per lane, respectively. (C) Western blot of peptides secreted by transduced PM-1 cells. PM-1 cells were transduced with 2xC46 Furin opt to different percentages as indicated. 2x10⁴ cells were seeded in 100 µl fresh medium. After 24 h incubation supernatants containing secreted peptides were collected, deglycosylated, and analyzed in Western blot (8 µl per lane).

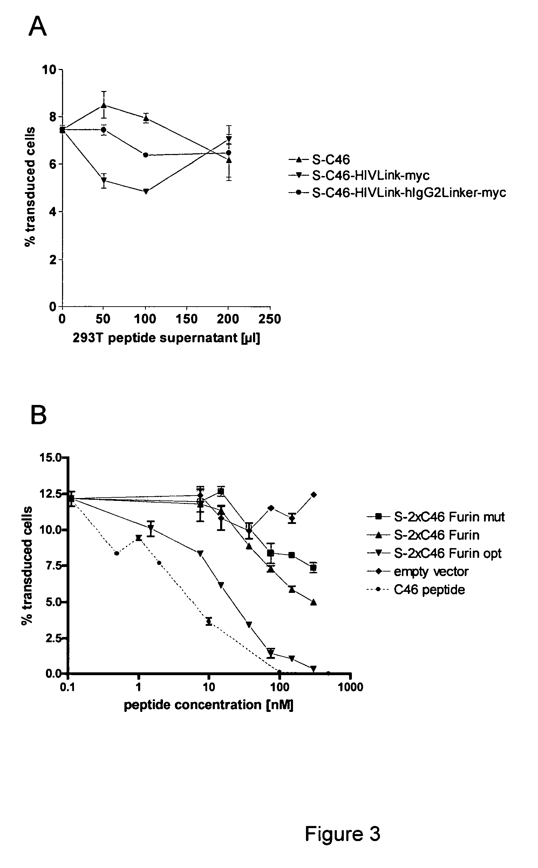

Figure 3: 2xC46 Furin opt shows antiviral activity in a single-round infection assay. PM-1 cells were transduced with replication-incompetent lentiviral vectors (pseudotyped with the HIV-1 JRFL envelope glycoprotein, and encoding eGFP) in the presence of serial dilutions of supernatants of transfected HEK 293T cells containing secreted peptides. Percentage of transduced eGFP positive cells was determined by flow cytometry. (A) Different volumes of cell culture supernatants, were used to analyze inhibitory activity of indicated constructs, final volume was 300 µl in each case. Same peptide supernatants as in Fig. 2A. (B) Concentrations of the secreted peptides in the cell culture supernatants were estimated by Western blot (Fig. 2B). As positive control synthetically produced C46 peptide was used to inhibit transduction of the cells by vector particles.

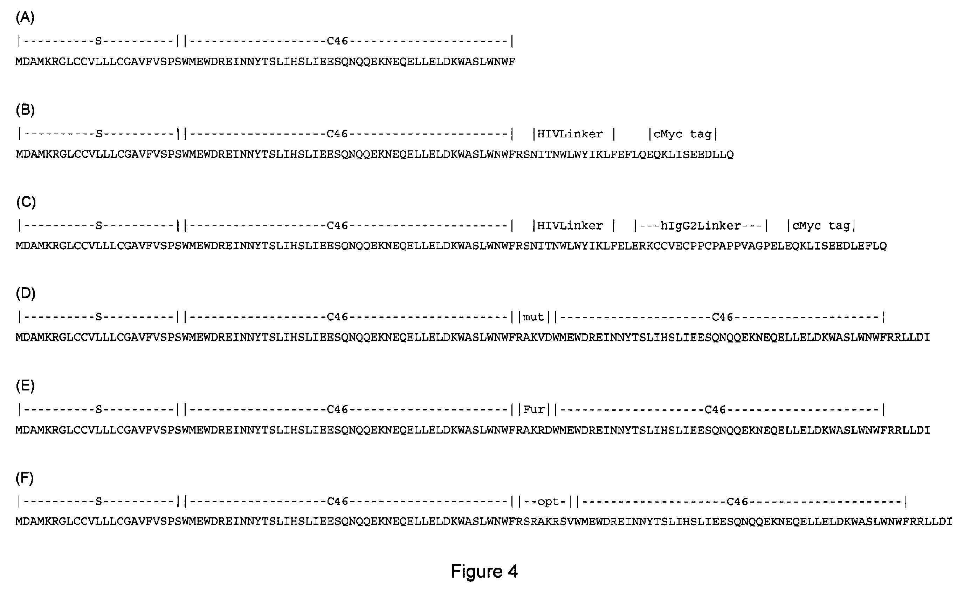

Figure 4: Sequences of transgenes used. (A) S-C46 (serial number M851), (B) S-C46-HIVLinker-myc (M852), (C) S-C46-HIVLinker-hIgG2Linker-myc (M808), (D) S-C46-Furin mut-C46 (M845), (E) S-C46-Furin-C46 (M818), (F) S-C46-Furin opt-C46 (M853). Fur, Furin cleavage site; mut, mutated furin cleavage site; opt, optimized furin cleavage site; S, signal peptide from human tissue type plasminogen activator (tPA).

### DETAILED DESCRIPTION OF THE INVENTION

### NUCLEIC ACID

In one aspect the invention relates to a nucleic acid of the general formula
5' - SP - EI1 - LINKER - EI2 - 3',
wherein
- 5': designates the 5' end of the nucleic acid sequence,
- 3': designates the 3' end of the nucleic acid sequence,
- SP: encodes a signal peptide,
- EI1: encodes an HIV entry inhibitory peptide,
- EI2: encodes an HIV entry inhibitory peptide, and
- LINKER: encodes a linker between EI1 and EI2; and
wherein the linker is a cleavable linker or a non-cleavable, flexible linker.

Expression of the nucleic acid results in a secretable HIV entry inhibitory (antiviral) peptides as a dimer (EI1 and EI2 encode different peptides) or concatamer (EI1 and EI2 encode identical peptides) of two peptides connected by a linker.

### SP

"SP" encodes a signal peptide. The signal peptide targets the HIV entry inhibitory (antiviral) peptide of the invention through the endoplasmatic reticulum to the cell surface and hence allows secretion of the peptide from transfected or transduced cells. The signal peptide is derived from human, non-immunogenic proteins, preferably selected from the group consisting of sequences coding for signal peptides of cellular membrane proteins. For example, the signal peptide can be the signal peptide of interleukin 2 receptor (IL-2R), granulocyte macrophage colony stimulating factor receptor (GM-CSFR), (human) low affinity nerve growth factor receptor (LNGFR), human tissue-type plasminogen activator (tPA), or (murine) Igk.

It is preferred that the signal peptide of the invention has one of the sequences (SEQ ID NOs:1-3) listed in Table 1.

**Table 1: Signal peptides**

| | |
|---|---|
| (1) SP-tPA | MDAMKRGLCCVLLLCGAVFVSPS |
| (2) SP-LNGFR | MGAGATGRAMDGPRLLLLLLLGVSLGGA |
| (3) SP-mIgk | METDTLLLWVLLLWVPGSTGD |

### EI1 and EI2

"EI1" and "EI2" each encodes an HIV entry inhibitory (antiviral) peptide, wherein the peptides may be identical or different. Accordingly, in one embodiment, "EI1" and "EI2" encode identical antiviral peptides. In another embodiment, "EI1" and "EI2" encode different antiviral peptides.

In the context of the invention, an HIV entry inhibitory peptide is a peptide capable of preventing the HIV entry into the cell. The peptides of the invention may be derived from, for example, gp41 protein of HIV, gp120 protein of HIV, HIV receptor CD4 and HIV co-receptors CXCR4 and CCR5.

In the context of the invention, "HIV" includes HIV-1 and HIV-2 types. Further included are all natural HIV strains (quasispecies or isolates). In this respect, it is understood that HIV strains include all known HIV strains as listed in the Los Alamos National Laboratory HIV Sequence Database.

In one aspect, an HIV entry inhibitory peptide is a natural peptide derived from the gp41 protein of HIV, preferably HIV-1. The amino acid sequence of the gp41 protein from HIV-1 is represented in SEQ ID NO:32. In one embodiment, the peptide is a natural peptide derived from a region comprising the second heptad repeat of the gp41 protein. The second (C) heptad repeat corresponds to amino acid positions 112 to 154 in SEQ ID NO:32. The position of the heptad repeats can slightly vary in different HIV strains. It is preferred that the antiviral peptide comprises, or consists of the second heptad repeat region or a portion thereof. According to the invention, a portion of the heptad repeat region has a length of at least 10 amino acids, at least 15 amino acids, at least 20 amino acids, at least 25 amino acids, at least 30 amino acids, or at least 35 amino acids. In a preferred embodiment, the antiviral peptide comprises the sequence YTSLIHSLIEESQNQQEKNE (SEQ ID NO:4) from the second heptad repeat of the HIV-1 gp41 protein.

According to another embodiment, the antiviral peptide is a synthetic peptide whose sequence is based on the sequence of the natural gp41 protein. It is again preferred that the sequence of the synthetic antiviral peptide is derived from the sequence of the second heptad repeat of the HIV-1 gp41 protein. Further included are peptides that mimic the tertiary structure of the second heptad repeat and bind to the groove formed by the central heptad repeat coil-coil.

The antiviral peptide of the invention has a length of between 20 and 60 amino acids, between 25 and 55 amino acids, between 30 and 50 amino acids, or between 35 and 50 amino acids. It is preferred that the antiviral peptide is 34, 35, 36, 36, 37, 38, 39, 40, 41, 42, 43, 44, 45, 46, 47, or 48 amino acids long.

It is preferred that the antiviral peptide of the invention has one of the sequences (SEQ ID NOs:5-13) listed in Table 2 (the sequences are homology aligned).

**Table 2: HIV entry inhibitory peptides**

| | |
|---|---|
| (5) C46 | WMEWDREINNYTSLIHSLIEESQNQQEKNEQELLELDKWASLWNWF |
| (6) C46mutGlyco | WMEWDREINNYASLIHSLIEESQNQQEKNEQELLELDKWASLWNWF |
| (7) C34 | WMEWDREINNYTSLIHSLIEESQNQQEKNEQELL |
| (8) C36 | YTSLIHSLIEESQNQQEKNEQELLELDKWASLWNWF |
| (9) T-1249 | WQEWEQKITA-------LLEQAQIQQEKNEYELQKLDKWASLWEWF |
| (10) T-649 | WMEWDREINNYTSLIHSLIEESQNQQEKNEQELLEL |
| (11) SC35EK | WEEWDKKIEEYTKKIEELIKKSEEQQKKNEEELKK |
| (12) T-2635 | TTWEAWDRAIAEYAARIEALIRAAQEQQEKNEAALREL |
| (13)HIV-2EHO-C46 | WQQWERQVRFLDANITKLLEEAQIQQEKNMYELQELDKWASLWNWF |

It is preferred that at least one of EI1 and EI2 encodes C46.

With the exception of HIV-2EHO-C46, all peptides of Table 1 are derived from HIV-1 gp41. However, peptides from HIV-2 that correspond to these peptides, i.e., their amino acid residues have the corresponding positions in the respective gp41 amino acid sequence, are also within the scope of the invention. Similarly, the invention includes corresponding peptides from different HIV-1 and HIV-2 strains.

The invention further includes peptides derived from the peptides as described above by amino acid exchanges that improve the solubility or the inhibitory effect of the peptide.

The invention further includes antiviral peptides that are homologous to the antiviral peptides described above. In the context of the application, "homologous" means at least 60% identity, at least 70% identity, preferably 80% identity, more preferably 90% identity, even more preferably 95% identity.

### LINKER

"LINKER" between EI1 and EI2 encodes a linker that connects the two antiviral peptides that are expressed as a concatamer (EI1 and EI2 encode identical peptides), or a dimer (EI1 and EI2 encode different peptides). The linker confers flexibility or allows cleavage of the concatamer or dimer into monomeric peptides. Accordingly, the linker can be a cleavable or non-cleavable, flexible linker. The linker also contributes to the elongation of the secretable portion of the peptide encoded by the nucleic acid of the invention, and hence more efficient entry into the secretory pathway.

The linker has a length of between 4 and 40 amino acids, between 5 and 30 amino acids, between 6 and 20 amino acids, or between 7 and 10 amino acids. In particular, the length of the linker can be 5, 6, 7, 8, 9, 10, 11, 12, 13, 14, 15, 16, 17, 18, 19 or 20 amino acids.

In one embodiment, the linker is a cleavable linker. A cleavable linker is a linker containing an amino acid sequence cleavable by a protease, i.e. a protease cleavage site. However, the linker may as well comprise a mutated, elongated or otherwise altered protease cleavage site. Mutations and alteration can result in a change of the cleavage site properties, such as optimisation of cleavage by the protease. For example, the linker may comprise a furin or SKI-1 protease cleavage site defined by a consensus sequence.

It is preferred that a cleavable linker of the invention comprises one of the sequences (SEQ ID NOs:14-19) listed in Table 3.

**Table 3: Cleavable Linkers**

| | |
|---|---|
| (14) Furin 1 | RAKR |
| (15) Furin 2 | RGRR |
| (16) Furin opt | RSRAKRSV |
| (17) Furin GG | GGRGRRGG |
| (18) gp160Furin | PTKAKRRWQREKRAVGIG |
| (19) SKI-1 | RRLL |

It is highly preferred that the linker has the sequence of an elongated furin recognition site such as Furin opt.

In another embodiment, the linker is a non-cleavable, flexible linker. A non-cleavable, flexible linker is usually a short peptide without a protease cleavage site that confers flexibility to the peptide of the invention. Such linker can be derived, for example, from HIV gp41, the human immunoglobulin G (IgG), preferably IgG2, the human P-glycoprotein, the human replication protein A, or parathyroid hormone-related proteins. For example, the IgG2-derived linker provides for dimerization of the peptides EI1 and EI2 *via* its cysteine residues. Flexibility of the linker is ensured by its minimal length of ∼7-8 amino acids.

It is preferred that a non-cleavable linker of the invention comprises one of the sequences (SEQ ID NOs:20-23) listed in Table 4.

**Table 4: Non-cleavable Linkers**

| | |
|---|---|
| (20) IgG2 | ERKCCVECPPCPAPPVAGP |
| (21) HIV-1 gp41 | NITNWLWYIKLF |
| (22) (G4S)2 | GGGGSGGGGS |
| (23) (G4S)4 | GGGGSGGGGSGGGGSGGGGS |

It is highly preferred that the linker has the sequence (G4S)2 or (G4S)4.

Examples of proteins encoded by the nucleic acid molecules generated in the course of the invention are given in Figure 4A-F (SEQ ID NOs:24-29). It is preferred that the nucleic acid of the invention encodes the amino acid sequence SEQ ID NO:29 or the following sequence (SEQ ID NO:30) derived therefrom and lacking six C-terminal amino acids.

MDAMKRGLCCVLLLCGAVFVSPSWMEWDREINNYTSLIHSLIEESQNQQEKNEQELLELD-
KWASLWNWFRSRAKRSVWMEWDREINNYTSLIHSLIEESQNQQEKNEQELLELDKWASL-
WNWF

It is highly preferred that the nucleic acid of the invention has the following sequence (SEQ ID NO:31), which encodes SEQ ID NO:30.

ATGGATGCAATGAAGAGAGGGCTCTGCTGTGTGCTGCTGCTGTGTGGAGCAGTCTTCGTTT-
CGCCCAGCTGGATGGAGTGGACCGCGAGATCAACAACTACACCAGCCTGATCCACAGCCT-
GATCGAGGAGAGCCAGAACCAGCAGGAGAAGAACGAGCAGGAGCTGCTGGAGCTGGACAAG-
TGGGCCAGCCTGTGGAACTGGTTCAGGAGCCGCGCCAAGAGGAGCGTGTGGATGGAGTGG-
GACCGCGAGATTAACAACTACACCAGCCTGATCCACAGCCTGATCGAGGAGAGCCAGAAC-
CAGCAGGAGAAGAACGAGCAGGAGCTGCTGGAGCTGGACAAGTGGGCCAGCCTGTGGAACT-
GGTTCTAG

### VECTOR

In another aspect, the invention relates to a vector containing the nucleic acid of the invention. The vector of the invention is any vector suitable for delivering the nucleic acid of the invention into cells by means of transduction or transfection. For example, the vector is a retroviral vector, a lentiviral vector, an Epstein-Barr virus (EBV) vector, a non-integrating viral vector such as an adenoviral vector, or a non-viral vector such as a plasmid. In one embodiment, a viral vector is a self-inactivating vector (SIN vector).

The vector of the invention preferably contains a packaging signal, a post-transcriptional regulatory element, a splice donor and a splice acceptor. Example of a packaging signal is the packaging signal ψ for packaging of a retroviral vector. Example of a post-transcriptional regulatory element is the woodchuck hepatitis virus posttranscriptional regulatory element (wPRE).

Examples of vectors of the invention are given in Figure 1.

### CELLS

In a further aspect, the invention relates to cells transduced or transfected with the vector of the invention. The cells of the invention are cells of the lymphocyte lineage such as B and T cells, haematopoietic stem or progenitor cells, or mesenchymal stem or stromal cells.

In one embodiment, the cells of the invention are T cells. T cells are preferred for safety reasons, because leukemogenesis as a result of retroviral insertional mutagenesis has never been reported in T cells. It is further preferred that the T cells are isolated from an HIV-infected patient to be treated, i.e. allogenic T cells.

### METHODS

In a further aspect, the invention relates to a method for in vitro transfection of cells of the lymphocyte lineage, haematopoietic stem or progenitor cells, or mesenchymal stem or stromal cells, with the vector of the invention.

According to the invention, the cells are transfected by a non-viral vector such as a plasmid or a non-integrating viral vector such as adenoviral vector, or are transduced by a stably integrating vector such as a retroviral or EBV vector, according to methods known in the art.

### MEDICAL USE

In a further aspect, the invention relates to the use of the vector of the invention for the preparation of a pharmaceutical composition for the treatment of patients infected with the human immunodeficiency virus.

In a further aspect, the invention relates to the use of the cells of the invention for the preparation of a pharmaceutical composition for the treatment of patients infected with the human immunodeficiency virus.

In one embodiment, T cells isolated from a patient to be treated are transfected or transduced with the vector of the invention and are implanted into the patient.

Finally, the invention relates to a pharmaceutical composition comprising the vector or the cell of the invention. The pharmaceutical composition contains a pharmaceutically acceptable carrier, and may further contain components common to pharmaceutical compositions.

The invention is illustrated by the following examples.

### EXAMPLES

### Constructs and methods for secretion of HIV-1 entry inhibitory peptides

The aim of the invention was the development of methods that would allow the improved secretion of HIV-1 entry inhibitory peptides by human cells in order to be applied for a novel gene therapy approach.

First, different linkers were analyzed for the elongation of the inhibitory peptides, some of which resulted in greatly enhanced secretion. However, these elongated peptides no longer showed antiviral activity in a single-round infection assay with replication-incompetent lentiviral particles.

Peptide dimers or concatamers were then constructed in which two antiviral peptides are connected by a linker that may contain a protease cleavage site, thus resulting in monomeric peptide fragments after protease cleavage past initiation of the secretory pathway. Peptide concatamers were secreted in high amounts by transfected and transduced cells, and some of the concatamer constructs exhibited distinct antiviral activity in a single-round infection assay.

### Materials and methods

Plasmids: All retroviral vectors encoding secretable HIV-1 entry inhibitory peptides are derivatives of MP91 (Schambach et al., Mol Ther 2000). This vector contains LTRs from myeloproliferative sarcoma virus (MPSV) and a modified untranslated leader derived from murine embryonal stem cell virus (MESV). The leader is devoid of *gag*-sequences and potential start codons (ATG) prior to the transgene, but contains an additional viral splice donor (SD) and a splice acceptor (SA). An enlarged multiple cloning site (MCS) was introduced into the NotI site of MP91, and a woodchuck hepatitis virus posttranscriptional regulatory element (wPRE) was inserted. The transgenes encoding secretable HIV-1 fusion inhibitory peptides were cloned according to state of the art molecular biology methods and integrated into the newly established MCS.

Cells and culture conditions: HEK 293T cells and the T cell line PM-1 were maintained in DMEM and RPMI respectively, supplemented with 5% fetal bovine serum, 100 units/ml penicillin, 100 µg/ml streptomycin, and 2 mM L-glutamine.

Virus production and titration: Viral vector supernatants were produced in HEK 293T cells. 6x10⁶ cells were plated on 9.4 cm dishes 24 h prior to transfection. For transfection, the medium was changed and 25 µM chloroquine was added. 7.5 µg retroviral vector DNA was transfected by the calcium phosphate precipitation method. To generate GaLV pseudotyped retroviral particles 2 µg pALF-GaLV CM (GaLV CM expression plasmid; Stitz et al., Virology 2000) and 12.5 µg M57-DAW (MuLV gag/pol expression plasmid; Schambach et al., Gene Ther 2006) were transfected in addition. For the production of lentiviral vectors encoding eGFP, and pseudotyped with the HIV-1 JRFL envelope glycoprotein 7.5 µg pHR'SIN-cPPT-SEW-new (SIN lentiviral vector encoding eGFP), 12.5 ug pCMV-dR8.91 (lentiviral gag/pol expression plasmid), and 1 µg M118 (HIV-1 JRFL expression plasmid) were co-transfected. Medium was changed after 6-8 h incubation. Supernatants containing viral particles were collected 36-60 h after transfection and filtered through 0.45-µm filters. Lentiviral vector particles were concentrated by ul-tracentrifugation on 20% sucrose in PBS for 2 h at 25,000 rpm and 4°C. Titration of vector supernatants was done by transduction of PM-1 cells by centrifugation for 1 h at 1000 g and 31°C. In the case of lentiviral vectors expressing eGFP, vector titers were determined by transducing predefined numbers of PM-1 cells with serial dilutions of vector supernatant and analyzing the percentage of eGFP positive cells by flow cytometry. For retroviral vectors expressing secreted peptides transduced cells were analyzed by intracellular immunostaining for the peptide of interest. Intracellular staining with the phycoerythrin-conjugated c2F5 antibody was performed using the Fix&Perm^{®} cell permeabilization reagents (Caltag Laboratories) according to the manufacturer's protocol and subsequent flow cytometry analysis.

Analysis of peptide secretion in transiently transfected HEK 293T cells: 6x10⁶ HEK 293T cells were plated on 9.4 cm dishes 24 h prior to transfection. For transfection, the medium was changed and 25 µM chloroquine was added. 10 µg of retroviral expression vector DNA encoding for the respective secretable peptide, and 10 µg of empty vector DNA were transfected using the calcium phosphate precipitation method. Medium was exchanged for chloroquine-free medium after incubation for 6-8 h. Cell culture supernatants were harvested 36-48 h post transfection and filtered through 0.22-µm filters. For the production of cell extracts, 1x10⁷ cells were trypsinated, washed with PBS, and subsequently lysed in 200 µl ice-cold lysis buffer (50 mM HEPES, pH 7.5; 150 mM NaCl; 1% Triton X-100; 2% aprotinin; 2 mM EDTA, pH 8.0; 50 mM sodium fluoride; 10 mM sodium pyrophosphate; 10% glycerol; 1 mM sodium vanadate; 2 mM Pefabloc^{®}SC). After 30 minutes incubation on ice with regular vortexing, cell debris was removed by centrifugation at 13,000 rpm for 10 minutes.

Analysis of peptide secretion in stably transduced PM-1 cells: PM-1 cells were transduced with retroviral vector supernatants for expression of the respective inhibitory peptides. 1x10⁵ cells were seeded per well of a 24-well plate and transduced with the desired amount of retroviral vector supernatant by centrifugation for 1 h at 1000 g and 31°C. Transduction efficiencies were determined by intracellular staining and flow cytometry as described above. Predefined numbers of stably transduced cells were seeded in fresh media and incubated for defined time periods (24-48 h). Cell culture supernatants were collected and either filtrated through 0.22-µm filters or centrifuged for 10 minutes at 13,000 rpm to remove cells and debris. Production of cell extracts was done as described above.

Western Blot: For analysis by Western blot cell culture supernatants and cell extracts were deglycosylated by N-Glycosidase F treatment. 50 µl of cell culture supernatant or cell extract were incubated with 100-200 units N-Glycosidase-F (PNGase F, New England Biolabs) over night at 37°C. Western blotting was done under reducing conditions following standard procedures. Proteins were electrophoretically transferred to nitrocellulose (0.45µm, BioRad) or PVDF membranes (Amersham Biosciences). Membranes were blocked with 5% dry milk in PBS containing 0.1% Tween-20 overnight at 4°C, and stained with the c2F5 antibody recognizing an epitope within the gp41-derived peptide sequence (Polymun; diluted 1:10,000 in 5% dry milk in PBS containing 0.1% Tween-20), and a horseradish peroxidase-conjugated anti-human IgG from goat (Dianova; diluted 1:10,000 in 5% dry milk in PBS containing 0.1% Tween-20). Detection was performed using enhanced chemiluminescence (Amersham Biosciences) according to the manufacturer's instructions.

Single-round infection assay: PM-1 cells were transduced with lentiviral vector particles pseudotyped with the HIV-1 JRFL envelope glycoprotein, and encoding eGFP, in the presence of rising concentrations of HEK 293T cell culture supernatants containing secreted peptides to determine the inhibitory activity of secreted peptides. 2x10⁴ PM-1 cells were plated per well of a 96-well plate, and transduced to 10-20% with the lentiviral particles in the presence of secreted peptides, by centrifugation for 1 h at 1000 g and 31°C. The percentage of transduced cells (eGFP positive) was determined by flow cytometry after incubation at 37°C for 4 days.

### Results

Retroviral vectors for the expression of secretable entry inhibitory peptides for HIV-1 gene therapy were developed (Fig. 1). HIV-1 entry inhibitory peptides, e.g. C46, were suspected to be too short for entry into the secretory pathway, which was confirmed in our initial experiments (Fig. 2A). In the construct S-C46 the C46 peptide is linked to a signal peptide for secretion, but although S-C46 peptide could be verified in cell extracts of transfected HEK 293T cells by Western blot, no secretion of the peptide into the cell culture supernatant was detectable. We therefore analyzed different linkers for the elongation of the inhibitory peptides, in order to achieve secretion. In the construct S-C46-HIVLinker-myc, the original C46 sequence was elongated by the HIVLinker derived from HIV-1 gp41 and a c-Myc tag. However, the elongated version of C46 was hardly expressed in detectable amounts in the cells, and no secreted peptide was observed. Further elongation with a linker from human IgG2, which induced dimerization of the peptides via its cysteine residues, resulted in increased expression and considerable secretion of peptides by transfected HEK 293T cells. We therefore examined the inhibitory activity of the cell culture supernatants containing secreted peptides (or no peptides in case of S-C46 and S-C46-HIVLinker-myc) in a single-round infection assay with replication-incompetent lentiviral particles pseudotyped with HIV-1 JRFL envelope glycoproteins. As expected, supernatants without detectable amounts of secreted peptide showed no antiviral activity (Fig. 3A).

But despite containing considerable amounts of peptide, supernatant from cells transfected with S-C46-HIVLinker-hIgG2Linker-myc did not inhibit entry of the viral particles, probably due to multimerization of the peptides.

In the next step, peptide concatamers were designed in which two antiviral C46 peptides were connected by a linker, containing a furin protease cleavage site with the consensus sequence RAKR (Furin), or a mutated version of this sequence motif (RAKV, Furin mut), which is non-cleavable. These constructs were considered to be long enough to allow entry into the secretory pathway and thus secretion, and it was assumed, that cleavage by furin protease would convert the peptide dimers into monomers. Both peptide concatamers S-2xC46-Furin and S-2xC46-Furin mut, were secreted in high amounts by transfected HEK 293T cells (Fig. 2B), but showed only minor antiviral activity in single-round infection assay (Fig. 3B).

Consequently, to obtain secretable peptides with improved inhibitory activity, optimization of the linker sequence was considered. The linker motif RAKR of the original construct S-2xC46-Furin was thus elongated to RSRAKRSV (Furin opt). The new construct S-2xC46-Furin opt was secreted by transfected HEK 293T and transduced PM-1 cells in considerable amounts (Fig 2B, C). Interestingly, elongation of the linker sequence resulted in a substantially improved antiviral activity of the peptides, which was almost as good as that of synthetically produced C46 peptide in single-round infection assay (Fig. 3B). Thus, the S-2xC46-Furin opt vector allows secretion of peptides, that are highly efficient HIV entry inhibitors, and therefore might be applied for a novel HIV-1 gene therapy approach.

### LITERATURE

Egelhofer, M., G. Brandenburg, et al. (2004). "Inhibition of human immunodeficiency virus type 1 entry in cells expressing gp41-derived peptides." J Virol 78(2): 568-75.
Eskridge, E. M. and D. Shields (1983). "Cell-free processing and segregation of insulin precursors." J Biol Chem 258(19): 11487-91.
Lipp, J., B. Dobberstein, et al. (1987). "Signal recognition particle arrests elongation of nascent secretory and membrane proteins at multiple sites in a transient manner." J Biol Chem 262(4): 1680-4.
Schambach, A., D. Mueller, et al. (2006). "Overcoming promoter competition in packaging cells improves production of self-inactivating retroviral vectors." Gene Ther 13(21): 1524-33.
Schambach, A., H. Wodrich, et al. (2000). "Context dependence of different modules for posttranscriptional enhancement of gene expression from retroviral vectors." Mol Ther 2(5): 435-45.
Stitz, J., C. J. Buchholz, et al. (2000). "Lentiviral vectors pseudotyped with envelope glycoproteins derived from gibbon ape leukemia virus and murine leukemia virus 10A1." Virology 273(1): 16-20.
van Lunzen, J., T. Glaunsinger, et al. (2007). "Transfer of autologous gene-modified T cells in HIV-infected patients with advanced immunodeficiency and drug-resistant virus." Mol Ther 15(5): 1024-33.
von Laer, D., C. Baum, et al. (2007). "Gene Therapeutic Approaches for Immune Modulation in AIDS." Anti-Inflammatory and Anti-Allergy Agents in Medicinal Chemistry 6(2): 121-140.
von Laer, D., S. Hasselmann, et al. (2006). "Gene therapy for HIV infection: what does it need to make it work?" J Gene Med 8(6): 658-667.
von Laer, D., S. Hasselmann, et al. (2006). "Impact of gene-modified T cells on HIV infection dynamics." J Theor Biol 238(1): 60-77.

## Claims

1. A nucleic acid of the general formula
5' - SP - EI1 - LINKER - EI2 - 3',
wherein
| | |
|---|---|
| 5' | designates the 5' end of the nucleic acid sequence, |
| 3' | designates the 3' end of the nucleic acid sequence, |
| SP | encodes a signal peptide, |
| EI1 | encodes an HIV entry inhibitory peptide, |
| EI2 | encodes an HIV entry inhibitory peptide, and |
| LINKER | encodes a linker between EI1 and EI2; and |
wherein the linker is a cleavable linker or a non-cleavable, flexible linker.

2. The nucleic acid of claim 1, wherein EI1 and EI2 encode identical HIV entry inhibitory peptides.

3. The nucleic acid of claim 1, wherein EI1 and EI2 encode different HIV entry inhibitory peptides.

4. The nucleic acid of claims 1 to 3, wherein the HIV entry inhibitory peptide has a length of between 20 and 60 amino acids.

5. The nucleic acid of claims 1 to 4, wherein the HIV entry inhibitory peptide has an amino acid sequence comprising a sequence selected from the group consisting of SEQ ID NO:4 to SEQ ID NO:13.

6. The nucleic acid of claims 1 to 5, wherein the HIV entry inhibitory peptide has an amino acid sequence selected from the group consisting of SEQ ID NO:4 to SEQ ID NO:13.

7. The nucleic acid of claims 1 to 6, wherein the signal peptide is selected from the group comprising a signal peptide of interleukin 2 receptor (IL-2R), granulocyte macrophage colony stimulating factor receptor (GM-CSFR), (human) low affinity nerve growth factor receptor (LNGFR), human tissue-type plasminogen activator (tPA), and (murine) Igk.

8. The nucleic acid of claims 1 to 7, wherein the linker has a length of between 4 and 40 amino acids.

9. The nucleic acid of claims 1 to 8, wherein the linker is a cleavable linker having a sequence comprising a sequence selected form the group consisting of SEQ ID NO:14 to SEQ ID NO:19.

10. The nucleic acid of claim 9, wherein the linker has a sequence selected from the group consisting of SEQ ID NO:14 to SEQ ID NO:19 and sequences having 1-5 additional amino acids at one or both ends of said sequences.

11. The nucleic acid of claims 1 to 8, wherein the linker is a non-cleavable, flexible linker having a sequence comprising a sequence selected form the group consisting of SEQ ID NO:20 to SEQ ID NO:23.

12. The nucleic acid of claims 1 to 11, wherein the nucleic acid encodes a protein having a sequence comprising SEQ ID NO:30.

13. The nucleic acid of claim 13, wherein the nucleic acid encodes the protein having the sequence SEQ ID NO:30.

14. A vector comprising the nucleic acid sequence of claims 1 to 13.

15. The vector of claim 14, wherein the vector is selected from the group consisting of a retroviral vector, a lentiviral vector, an Epstein-Barr virus (EBV) vector, an adenoviral vector, and a non-viral vector.

16. A cell transfected or transduced with the vector of claim 14 or 15.

17. The cell of claims 16, wherein the cell is a cell of the lymphocyte lineage, a haematopoietic stem or progenitor cell, or a mesenchymal stem or stromal cell.

18. An *in vitro* method for transfection or transduction of a cell comprising transfecting or transducing a cell with the vector of claims 14 or 15, wherein the cell is a cell of the lymphocyte lineage, a haematopoietic stem or progenitor cell, or a mesenchymal stem or stromal cell.

19. Use of the vector of claim 14 or 15 for the preparation of a pharmaceutical composition for the treatment of patients infected with HIV.

20. Use of the cell of claim 16 or 17 for the preparation of a pharmaceutical composition for the treatment of patients infected with HIV.

21. A pharmaceutical composition comprising the vector of claim 14 or 15 and a physiologically acceptable carrier.

22. A pharmaceutical composition comprising the cell of claim 16 or 17 and a physiologically acceptable carrier.
